# EUROPEAN PATENT APPLICATION

(11) **EP 3 000 471 A1**
(43) Date of publication of application: **30.03.2016**
(21) Application number: 14186723.4
(22) Date of filing: 28.09.2014
(51) Int. Cl.: A61K 31/683, A61K 31/7034, C07H 15/20, C07F 9/10, A61P 35/00, A61P 33/02, A61K 35/15, A61P 33/00, A61P 31/00

(54) **Novel immunostimulatory molecules**

(71) Applicant: Bernhard-Nocht-Institut für Tropenmedizin, 20359 Hamburg (DE); Forschungszentrum Borstel, 23845 Borstel (DE)
(72) Inventor: Bernin, Hannah, 22559 Hamburg (DE); Lotter, Hannelore, 21521 Wohltorf (DE); Gonzalez-Roldan, Nestor, 23795 Negernbötel (DE); Tannich, Egbert, 22399 Hamburg (DE); Fujimoto, Yukari, 212-0032 Kanagawa (JP); Fukase, Koichi, 560-0033 Osaka (JP)
(74) Representative: UEXKÜLL & STOLBERG

(57) **Abstract**

The present invention relates to novel immunostimulatory molecules which are derived from the intestinal protozoan *Entamoeba histolytica.* The compounds have been found to be useful for enhancing and/or inducing an immune response in a subject in need thereof. Specifically, the compounds have been found to be useful for the treatment of cancer diseases, such as breast cancer, and parasitic diseases, such as leishmaniasis. The invention also provides pharmaceutical compositions comprising the novel compounds.

## Description

The present invention relates to novel immunostimulatory molecules which are derived from a glycolipid molecule of the intestinal protozoan *Entamoeba histolytica.* The compounds have been found to be useful for enhancing and/or inducing an immune response in a subject in need thereof. Specifically, the compounds have been found to be useful for the treatment of cancer diseases, such as breast cancer, and intracellular infections, such as leishmaniasis. The invention also provides pharmaceutical compositions comprising the novel compounds.

### BACKGROUND OF THE INVENTION

A number of diseases involve pathological processes that interfere with an effective immune response. For example, certain diseases are known to involve immunomodulatory mechanisms that actively suppress an immune response that would ameliorate or prevent the spread of the disease. In addition, an exhaustion of the adaptive immune response can be observed in chronic diseases like hepatitis B infections. It is known in the art that immunostimulatory molecules can be used for the activation of antigen presenting cells (APC) or natural killer T cells (NKT). In this way, these molecules can be used for inducing or enhancing an immune response.

NKT cells are heterogeneous cell populations, but the majority of these cells express an invariant Va14-Ja18 T cell receptor (TCR). These cells are referred to in the scientific literature as invariant NKT (iNKT) cells. Upon ligation of their TCR, iNKT cells can produce large amounts of different cytokines, including the pro-inflammatory IFN-γ, but also anti-inflammatory cytokines like IL-4, IL-13 or IL-10, which is believed to induce the development of an immune response. The far most studied iNKT cell antigen is the glycolipid α-galactosylceramide (α-GalCer), a marine sponge glycolipid, which is a potent CD 1 d-restricted agonist widely used for *in vitro* and *in vivo* experiments to decipher iNKT cell function. In recent years, it has been shown that iNKT cells can be activated directly by recognition of microbial glycolipids presented by CD1d or, indirectly by soluble mediators such as IL-12 and/or by recognition of endogenous ligands, both provided by dendritic cells (DCs) stimulated via Toll-like receptors (TLRs). See Laurent et al. 2014 and Blum et al. 2012. In addition to α-GalCer, diverse natural CD1d ligands that stimulate iNKT cells have been identified in microorganisms (Neumayr et al. 2013; Sosa et al. 2013; Barros et al. 2013). Accordingly, mice lacking iNKT cells have been shown to possess an increased susceptibility to various bacterial, fungal, and parasitic infections (Sosa et al. 2013; Barros et al. 2013).

Despite the progress that has been made in recent years, there is a need for novel immunostimulatory molecules that can be specifically used for the treatment of diseases that benefit from the enhancement of an immune response. EhLPPG is a glycolipid molecule that was isolated from the membrane of *Entamoeba histolytica* trophozoites and that was shown to have immunostimulatory properties. EhLPPG stimulates antigen presenting cells (APC, macrophages, dendritic cells) either by binding to TLRs or through the uptake into endosomes. Binding via TLRs leads to stimulation of the APCs through IL-12p70 production. APCs stimulated in this way are able to induce pro-inflammatory cytokines. The uptake of the EhLPPG molecule by APCs results in the loading of EhLPPG on CD1d molecules in so called "late endosomes". EhLPPG-CD1d complexes are translocated onto the surface of APCs resulting in the activation of NKT cells. The activated NKT cells rapidly produce large amounts of cytokines which modulate the adaptive immune response.

So far, however, the use of EhLPPG has only been shown to be effective in amebic liver abscess (Lotter at al. 2009). Furthermore, the use of EhLPPG is restricted by its limited availability. Only small amounts of EhLPPG can be isolated from the membrane of E. histolytica trophozoites by employing elaborative isolation procedures. Therefore, there is a need for new compounds that circumvent the limitations associated with the native molecule.

### DETAILED DESCRIPTION OF THE INVENTION

The present invention provides compounds and methods that can be used for inducing and/or enhancing immune responses in a patient or in isolated cells or cell cultures. It was found in the course of the present invention that EhLPPG can be used as an active agent in the treatment of intracellular infections caused by bacterial or protozoan parasites, such as protozoa of the genus *Leishmania.* It was also found that EhLPPG is therapeutically active in the treatment of cancer, in particular breast cancer. The present invention for the first time provides evidence that EhLPPG reduces the number of infected cells, such as infected macrophages, in a patient suffering from leishmaniasis. The experiments described herein demonstrate that tumor growth in mice into which tumor cells had been implanted can be reduced by (i) obtaining APCs from said mice, (ii) incubating the APCs with EhLPPG, and (c) reinjecting the APCs into said mice.

It is also disclosed in the present application that the immunostimulatory activities of EhLPPG are not restricted to the complete glycolipid conjugate which occurs in the membrane of *E. histolytica.* In contrast, it was found that the inositol phospholipid moiety of the GPI anchor (referred to herein as EhPI) represents the immunoactive part of the EhLPPG molecule which is responsible for inducing the immune response against cancer or *Leishmania* infected cells. From native EhLPPG, two GPI anchors can be isolated which are referred to herein as EhPIa und EhPIb. The natural GPI anchor EhPIa comprises two different forms, one containing a C28 saturated fatty acid (referred to herein as C28:0-EhPIa which makes up to 84% of EhPIa) and one containing a C30 saturated fatty acid (referred to as C30:1-EhPIa which makes up to about 16% of EhPIa). The ratio might vary in different preparations of natural EhPIa. EhPIb also comprises two structural forms which are referred to as C28:0-EhPIb, which makes up to 80% of EhPIb, and C30:1-EhPIb, the latter of which makes up to about 20% of EhPIb. Again, the specific ratio may slightly vary between different preparations of natural EhPIb.

The inventors have designed synthetic analogues, C30-EhPIa-cis, C30-EhPIb-cis, C30-EhPIb-trans and C28-EhPIb, that differ from EhPIa or EhPIb in the position and length of the fatty acid chains. It is demonstrated that these synthetic analogs exert the same activity as EhLPPG when used in an iNKT cell assay. Accordingly, the invention also contemplates these analogs for the treatment of cancer and infections caused by intracellular parasites, in particular leishmaniasis.

The present invention in a first aspect relates to a compound having the structure of the following formula (I): 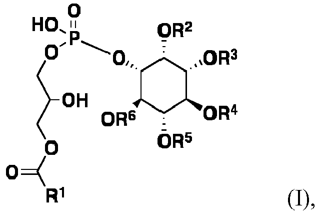 wherein
R¹ is Y or Z,
R², R³ and R⁵ are independently selected from -H, -C(O)Y, -C(O)Z, and groups of formula (II) 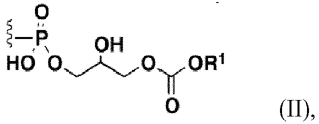 and
R⁴ and R⁶ are independently selected from -H, -C(O)Y, -C(O)Z, and groups derived from glycosides, preferably amino glycosides,
wherein
Y is CH₃(CH₂)ₘ-, and m = 0-35, and
Z is CH₃(CH₂)ₙ-(CH=CH)ₚ(CH₂)_{q}-, and n and q are independently 0-35, and p = 1-3
and pharmaceutically acceptable salts, solvates, prodrugs, and protected derivatives thereof,
for use in a method of treating a cancer disease or an intracellular parasitic infection caused by a parasite of the genus *Leishmania* in a subject in need thereof. The invention also includes the use of enantiomers and diastereomers of the compound of the above formula.

The invention therefore also relates to a method of treating a cancer disease or an intracellular infection, such as an infection caused by a parasite of the genus *Leishmania,* in a subject in need thereof, said method comprising administering to said subject a therapeutically effective amount of a compound having the structure of the following formula (I): 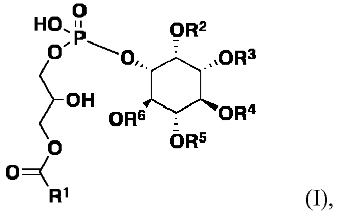 wherein
R¹ is Y or Z,
R², R³ and R⁵ are independently selected from -H, -C(O)Y, -C(O)Z, and groups of formula (II) 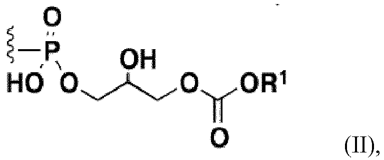 and
R⁴ and R⁶ are independently selected from -H, -C(O)Y, -C(O)Z, and groups derived from glycosides, preferably amino glycosides,
wherein
Y is CH₃(CH₂)ₘ-, and m = 0-35, and
Z is CH₃(CH₂)ₙ-(CH=CH)ₚ(CH₂)_{q}-, and n and q are independently 0-35, and p = 1-4,
and pharmaceutically acceptable salts, solvates, prodrugs, and protected derivatives thereof. The invention also includes enantiomers and diastereomers of the compound of the above formula.

Preferably, R¹ is -Z, and R³ and R⁵ are each -H.

In a preferred embodiment, Z is such that it is derived from monounsaturated fatty acids of the type ZCOOH. Preferred are *cis*-monounsaturated fatty acids ZCOOH.

Furthermore, it is preferred that ZCOOH is in the range C20:1 to C40:1, and preferred are C24:1 to C36:1, e.g. C26:1, C28:1, C30:1, C32:1, and C34:1, in particular it is preferred that Z is derived from ZCOOH being C30:1.

Preferably, R² is selected from -H and -C(O)Y (and is more preferably -C(O)Y), and R³ and R⁵ are each -H.

In a preferred embodiment of Y, m is 10-33, more preferably 10-31, in particular 12-18, such as most preferably 15. In other words, Y is most preferably derived from YCOOH being C16 saturated fatty acid.

Furthermore, it is preferred that the group derived from glycosides, when present, is selected from
α -GlcN-,
Glc(1-2)Man(1-6)Man(1-4)-GlcN-, and
NH2(CH2)20-P(=O)(OH)-Glc(1-2)Man(1-6)Man(1-4)GlcN-.

It is also generally preferred that R3, R4, R5 and R6 are all -H. Alternatively, it is generally preferred that R4 and R6 are independently selected from H and groups derived from glycosides, in particular amino glycosides.

In a first most preferred embodiment, the compound of the invention is C30EhPlb-cis, wherein R1COOH is C30:1(cis), R2 is -C(O)C15H31, and R3 to R6 are each H.

In a second most preferred embodiment of the invention, R1COOH is C30:1(cis), R2 is -C(O)C15H31, R3 to R5 are each -H, and R6 is selected from
α -GlcN-,
Glc(1-2)Man(1-6)Man(1-4)-GlcN-, and
NH2(CH2)20-P(=O)(OH)-Glc(1-2)Man(1-6)Man(1-4)GlcN-.

In a third most preferred embodiment of the invention, the compound is EhPIa or EhPIb which can be illustrated by the following formulas:

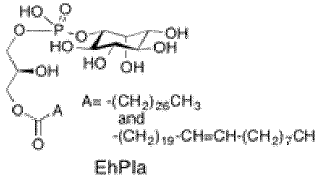

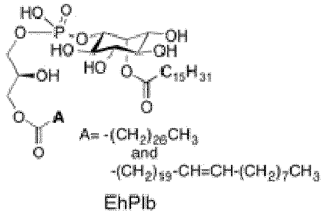

In another most preferred embodiment, the compound is selected from naturally occurring EhLPPG or from the following group of compounds:

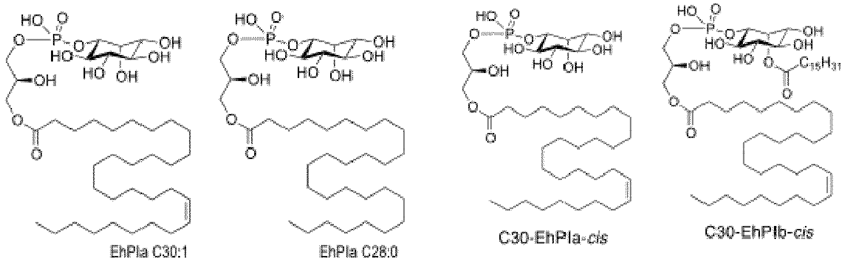

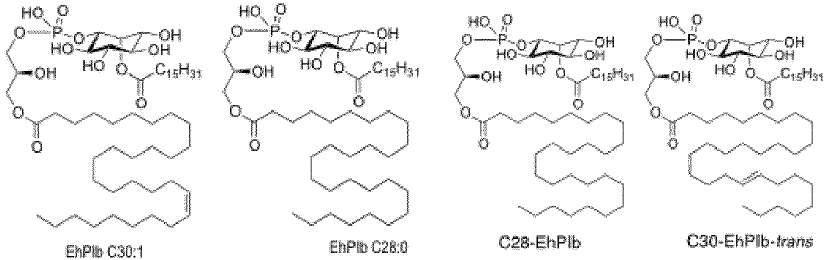

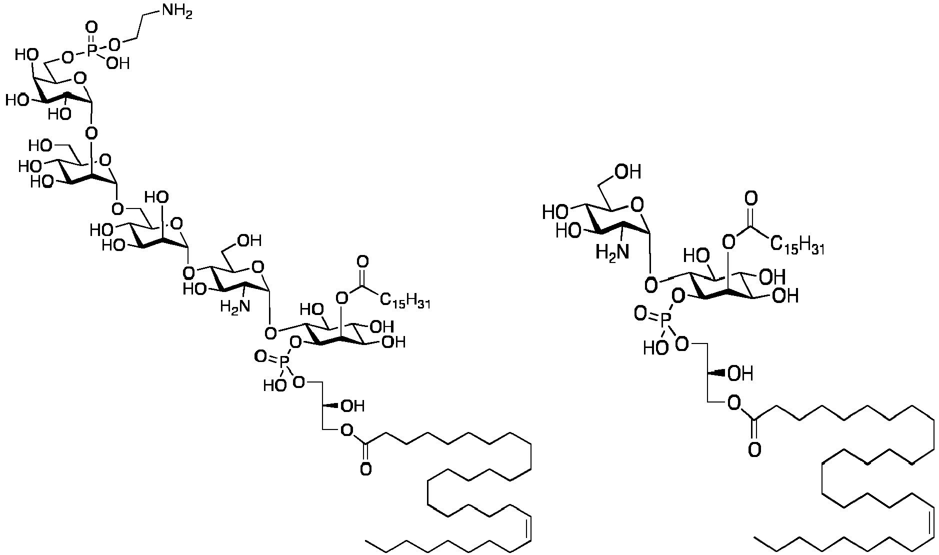

The invention also includes the use of enantiomers and diastereomers of the above compounds.

The subject to be treated with a compound of the present invention can in principle be any mammal, but it will preferably be a human. In a particular preferred embodiment, the subject to be treated according to the invention is a human patient afflicted with cancer, more specifically breast cancer. In a still further embodiment, the subject to be treated according to the invention is a human patient that suffers from an intracellular infection caused by a bacterial or protozoan parasite, such as a parasite of the genus Leishmania.

The experiments performed by the present inventors show that the administration of a compound as defined above is effective for inducing and/or enhancing an immune response which interferes with the growth and/or proliferation of cancer cells. The compounds of the invention are moreover effective in suppressing the spreading of cancer cells, thereby inhibiting the development of metastases. The immune response is induced and/or enhanced via activation of iNKT cells in the patient.

The cancer disease to be treated with the compounds of the invention is not particularly limited to certain cancer types but can include all different types of cancer that has been described in the prior art, including e.g. breast cancer, colon cancer, pancreatic cancer, stomach cancer, brain cancer, lung cancer, kidney cancer, liver cancer, ovarian cancer, cervical cancer, or thymus cancer.

In a particularly preferred embodiment, the cancer disease to be treated is breast cancer. For example, the breast cancer can be a carcinoma selected from the group of invasive ductal carcinoma, invasive lobular carcinoma, papillary carcinoma, mucinous carcinoma, medullary carcinoma and tubular carcinoma. The diagnosis and classification of cancers is well known in the art and discussed in numerous publications (see, for example, the publications provided by the World Health Organization, WHO/IARC Classification of Tumours).

According to the invention, it is also possible to administer more than one compound of the invention in order to treat or ameliorate the symptoms of the cancer disease or parasitic infection. For example, a treatment regimen can include the simultaneous or sequential administration of 2, 3, 4, 5, 6, or even more of the compounds having the formula as defined above. Apart from that, the administration of one or more compounds of the invention can be combined with the administration of other anti-cancer drugs which are common in the field of cancer treatment.

For example, where the treatment of cancer, such as breast cancer, is intended, it may be advantageous in terms of treatment efficiency to combine the administration of one or more of the compounds of the invention with the administration of one or more additional chemotherapeutic agents. Suitable agents that may be used in association with the compounds of the invention include, but are not limited to, alkylating agents; alkyl sulfonates; aziridines, such as Thiotepa; ethyleneimines; anti-metabolites; folic acid-analogues, such as methotrexate (Farmitrexat®, Lantarel®, METEX®, MTX Hexal®); purine analogues, such as azathioprine (Azaiprin®, AZAMEDAC®, Imurek®, Zytrim®), cladribin (Leu-statin®), fludarabin phosphate (Fulda®), mercapto purine (MERCAP®, Puri-Nethol®), pentostatin (Nipent®), thioguanine (Thioguanin-Wellcome®) or fludarabine; pyrimidine analogues, such as cytarabin (Alexan®, ARA-cell®, Udicil®), fluorouracil, 5-FU (Efudix®, Fluoroblastin®, Ribofluor®), gemcitabine (Gemzar®), doxifluridine, azacitidine, carmofur, 6-azauridine, floxuridine; nitrogen-lost-derivatives, such as chlorambucil (Leukeran®), melphalan (Alkeran®), chlornaphazine, estramustin, mechlorethamine; oxazaphosphorines, such as cyclophosphamide (CYCLO-cell®, Cyclostin®, Endoxan®), ifosfamide (Holoxan®, IFO-cell®) or trofosfamide (Ixoten®); nitrosureas, such as Benda-mustine (Ribomustin®), Carmustine (Carmubris®), Fotemustine (Muphoran®), Lomustine (Cecenu®, Lomeblastin®), Chlorozotocine, Ranimustine or Nimustine (ACNU®); hydroxy-ureas (Litalir®); taxens, such as docetaxel (Taxotere®), or paclitaxel (Taxol®); platinum-compounds, such as cisplatin (Platiblastin®, Platinex®) or carboplatin (Carboplat®, Ribocarbo®); sulfonic acid esters, such as busulfan (Myleran®), piposulfan or treosulfan (Ovastat®); anthracyclines, such as doxorubicin (Adriblastin®, DOXO-cell®), daunorubicin (Daunoblastin®), epirubicin (Farmorubicin®), idarubicin (Zavedos®), amsacrine (Amsidyl®) or Mitoxantrone (Novantron®); as well as derivates, tautomers and pharmaceutically active salts of the aforementioned compounds.

It will likewise be possible to combine the administration of one or more of the compounds of the invention with the administration of a therapeutic anti-cancer antibody. Several antibodies have been developed for treating cancer diseases, including trastuzumab (Herceptin®), bevacizumab (Avastin®), gemtuzumab (Mylotarg®), panitumumab (Vectibix®) or edrecolomab (Panorex®). The use of a tyrosine kinase inhibitor, such as sorafenib (Nexavar®) or sunitinib (Sutent®), is also contemplated herein.

The administration of one or more of the compounds of the invention may also be combined with other therapeutic approaches that have proven to be effective in the treatment of cancer, such as radiation. Radiation therapy or radiotherapy refers to the use of ionizing radiation for the targeted destruction of cancer cells or tissues. As used herein, radiation therapy includes various techniques for irradiating cancer tissue in a subject with high-energy radiation to destroy the cancer tissue. In the context of the invention, radiation therapy can include photon-based radiation, such as x-rays and gamma rays, and/or particle radiation, such as electron, carbon or proton beams. Preferably, radiation is performed as an external beam radiation therapy.

The overall dose of radiation that is administered to the subject during treatment will be in the range of 20-75 Gy, preferably 30-60 Gy and more preferably 45-60 Gy, for example, 45, 46, 47, 48, 49, 50, 51, 52, 53, 54, 55, 56, 57, 58, 59 or 60 Gy, administered over a time period of 3 to 8 weeks, e.g. over a time period of 3, 4, 5, 6, 7 or 8 weeks of therapy. In a preferred aspect, the daily dose of radiation that is administered is 1.8 Gy for a child or adolescent and 2.0 Gy for an adult. Radiation therapy can be applied either before, simultaneously with, or after the administration of the compounds of the invention.

In another aspect, the compounds of the invention are used for treating an intracellular infection. Intracellular infections have been described in the context with a number of different diseases. Both bacterial and protozoan parasites are known in the art. Bacterial parasites which infect human cells, such as macrophages and DCs, include bacteria of the genus Mycobacterium, such as M. tuberculosis, M. leprae, M. ulecrans, and M. marinum; bacteria of the genus Rickettsia, such as R. africanae, R. conorii, R. helvetica, and R. prowazekii; bacteria of the genus Orientia, such as O. tsutsugamushi; bacteria of the genus Chlamydia, such as C. pneumonia; bacteria of the genus Coxiella, such as C. burnetti; bacteria of the genus Francisella, such as F. tularensis; bacteria of the genus Listeria, such as L. monozytogenes; bacteria of the genus Legionella, such as L. pneumophila; and bacteria of the genus Brucella, such as B. melitensis.

Protozoan parasites that can be treated with the compounds of the invention in particular include organisms of the genus Leishmania. Leishmania are protozoan parasites that infect and grow within macrophages. The number of cases of the resulting disease, leishmaniasis, has constantly increased in the past years with about 2 million new cases each year. According to the World Health Organization, there are more than 12 million people infected worldwide, primarily in countries like Brazil, China, East Africa, and India.

Three main forms of leishmaniasis have been described: visceral leishmaniasis, also known as kalaazar, mucocutaneous leishmaniasis, and cutaneous leishmaniasis. Visceral leishmaniasis is the most severe form of the disease which leads to death if left untreated. Symptoms of visceral leishmaniasis include fever, weight loss, enlargement of the spleen and liver, and anaemia. Mucocutaneous leishmaniasis leads to partial or total destruction of mucous membranes of the nose, mouth and throat. Cutaneous leishmaniasis is the most common form of leishmaniasis. It causes ulcers on exposed parts of the body that leave life-long scars and serious disability. In most cases, cutaneous leishmaniasis heals spontaneously. However, in some patients the disease progresses and causes severe skin damages.

The examples of the present invention demonstrate that the compounds can be used for reducing the parasite burden in subjects suffering from leishmaniasis. Thus, in a preferred aspect, the compounds of the invention are used for the treatment of leishmaniasis. The disease can be caused by different parasites of the genus Leishmania. For example, the leishmaniasis to be treated according to the invention can be caused by L. aethiopica, L. donovani, L. infantum, L. major, L. chagasi, L. viannia, L. mexicana, L. amazonensis, L. tropica or L. tarentolae. In a particularly preferred aspect, the leishmaniasis to be treated is cutaneous leishmaniasis caused by L. aethiopica, L. major, L. mexicana, or L. amazonensis.

The specific amount of the compounds of the invention which is therapeutically effective in the subject suffering from, e.g. cancer or an intracellular infection, will depend on parameters, such as the mode of administration, the particular diseases, age, height, weight, health, and physical condition of the treated individual, and others. A therapeutically effective amount of a inositol phospholipid compound of the invention is typically sufficient to achieve a plasma concentration from about 0.05 µg/ml to about 250 µg/ml, preferably from about 0.1 µg/ml to 200 µg/ml, more preferably from about 1 µg/ml to about 100 µg/ml, and usually from about 10 µg/ml to about 50 µg/ml, e.g. 25 µg/ml. Stated differently, the weekly dose can vary from about 0.5 mg to about 200 mg per m2 of the patient's body surface. Preferably, the weekly dose of the compound will be from about 1-100 mg/m2, more preferably from 10-50 mg/m2, most preferably 20 to 25 mg/m2. The weekly dose is administered in one or more distinct administrations.

The inositol phospholipid compounds disclosed herein may be administered in any suitable form that does not interfere with its immunomodulatory activity. For example, the compounds may be administered orally in the form of tablets, capsules, granules, powder, liquids, and the like. The compounds may also be administered encapsulated in liposomes. Alternatively, the compounds may be formulated for being administered by intravenous injection or intravenous infusion. Compositions suitable for injection and/or infusion include solutions or dispersions and powders for the extemporaneous preparation of injectable solutions or dispersions. The composition for injection must be sterile and should be stable under the conditions of manufacturing and storage. Preferably, the compositions for injection and/or infusion also include a preservative, such as a chlorobutanol, phenol, ascorbic acid, thimerosal, and the like. For intravenous administration, suitable carriers may comprise physiological saline, bacteriostatic water, Cremophor EL™ (BASF) or phosphate buffered saline (PBS). Sterile solutions for injection and/or infusion can be prepared by incorporating the inositol phospholipid compound in the required amount in an appropriate solvent followed by filter sterilization.

In one preferred embodiment, the compounds disclosed herein are used for treating cutaneous leishmaniasis. According to the invention, where the treatment of cutaneous leishmaniasis is intended, it is preferred to administer the compound of the invention via a transdermal or transmucosal route, for example, by use of a cream, lotion, ointment, gel, or salve which provides for the topical delivery of the compound to the skin lesions, papules, nodules, or facial erythema which are evident on the skin of the patient to be treated. The compound of the invention can also be incorporated into an adhesive patch or medical strip which is placed on the skin lesion and provides for a sustained delivery of the drug to the skin.

Preferably, the cream, lotion, ointment, gel, or salve includes the one or more pharmaceutically active compounds of the invention in the form of liposomes, e.g. encapsulated in liposomes. Liposomes are artificial vesicles which consist of a phospholipid bilayer. Phospholipids normally comprise a polar "head" and two non-polar "tails". This structure results in the formation of a vesicular bilayer in which the polar heads align with an outer or inner aqueous phase and the non-polar tails align with each other. Liposomes have found wide use for the transdermal or transmucosal delivery of pharmaceutical active drugs. Liposomes provide for an improved penetration into deep skin layer which is advantageous in the treatment of cutaneous leishmaniasis. Liposomes are taken up by cells, in particular by macrophages, DCs and APCs. Accordingly, liposomes are able to deliver the active drug directly to the cells infected with the parasite.

According to the invention, liposomes which are preferred for use in the delivery of the pharmaceutical active compounds of the invention will have a size in the range of 30 to 10,000 nm, more preferably from 50 nm to 1,000, from 100 nm to 800, from 200 nm to 600, from 250 nm to 500, or from 300 nm to 400 nm. The liposomes can further be produced as multilamellar or unilamellar liposomes. Liposomes are commonly nontoxic, biodegradable and non immunogenic, because they can be made of naturally occurring components. The production of liposomes has been described in much detail in the prior art. See, for example, Arias et al. 2013 and the patent literature cited therein.

In another preferred embodiment, the compounds disclosed herein are used for treating visceral leishmaniasis. Currently, amphotericin B is the most commonly used drug for treating visceral leishmaniasis. However, amphotericin B is highly toxic. The simultaneous use of amphotericin B in combination with one of the compounds of the invention is contemplated which could result in a reduction of the amphotericin B dosage that is needed for treatment. Where the treatment of visceral leishmaniasis (kal-azar) or other intracellular infections is intended, the compound of the invention may likewise be formulated for administration by liposomes, as described above. In these cases, the liposomes will be administered via intravenous injection or infusion. Alternatively, the compound of the invention may be directly formulated for intravenous administration, e.g. without using liposomes. In a preferred aspect, the compounds are administered to a subject suffering from visceral leishmaniasis or another intracellular infection by intravenous infusion, more preferably by short-term infusion within less than 60 min, e.g. within 30 min, 20 min or 15 min.

Where the treatment of cancer, and in particular breast cancer, is envisaged the compounds of the invention can be administered via different routes, for example by intravenous administration, e.g. injection or infusion. However, according to a preferred embodiment, the compounds of the invention will be used for stimulating cells obtained from the patient in vitro, and the stimulated cells are then re-infused into the patient who suffers from the cancer disease. The cells will preferably be APCs, preferably APCs from peripheral blood. Upon presentation of a compound of the invention by APCs that express CD1d, iNKT are stimulated to produce large amounts of cytokines, including IFN-γ, which in turn results in the development of a pro-inflammatory immune response. The APCs are preferably dendritic cells which have been differentiated from blood monocytes according to standard protocols. According to another aspect, the compounds of the invention are administered in the form of liposomes, as described elsewhere herein for the treatment of cancer, in particular breast cancer.

In a still further aspect, the invention therefore refers to a method for preparing an autologous composition for inducing and/or enhancing an immune response in a patient, said method comprising
(a) separating CD14+ monocytes from a blood sample obtained from a cancer patient;
(b) differentiating said CD14+ monocytes into APCs;
(c) incubating the APCs with a compound of the invention as defined above; and
(d) optionally purifying the APCs.

In a first step, a blood sample is obtained from the cancer patient. The sample can be collected by venopuncture using a common syringe and a needle or butterfly needle of 21 to 23 g. The blood sample can be transferred into a blood sample tube for further processing. In a subsequent step, CD14+ monocytes are separated from a blood sample. For this, a density gradient centrifugation can be performed which separates the lymphocytes from red blood cells and neutrophils. CD14+ monocytes can then be isolated or enriched by using positive immunomagnetic selection against CD 14.

After isolation of the CD14+ monocytes, these cells are differentiated into APCs, in particular dendritic cells (DCs). Methods for differentiating CD 14+ monocytes into APCs, and in particular DCs are widely known in the art. For example, CD1d expressing immature DCs derived from monocytes can be obtained by culturing adherent monocytes with IL-4 and GM-CSF. Similarly, CD1d expressing mature DCs derived from monocytes can be obtained by culturing adherent monocytes with IL-4 and GM-CSF and subsequent maturation using IL-2 and α-GalCer, as described by Ishikawa et al. (2005) and Uchida et al. (2008).

After preparing the APCs, the cells are incubated for 3-4 hours with 10-100 µg/ml, more preferably 20-50 µg/ml of a inositol phospholipid compound of the invention, for example with EhPIa und EhPIb or one of the analogs C30-EhPIa-cis, C30-EhPIb-cis, C28-EhPIb and C30-EhPIb-trans disclosed herein. The so obtained product (1x10⁸ stimulated APCs) can be re-infused into the patient to be treated. The stimulated cells will be useful for eliciting an immune response against the cancer cells in the patient.

In still another aspect, the invention relates to an immunomodulatory compound having a structure selected from the group of: 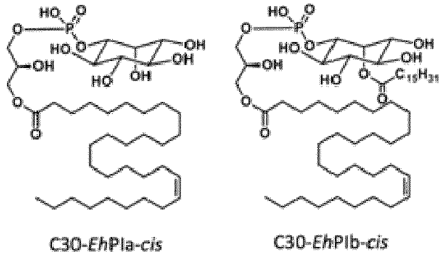 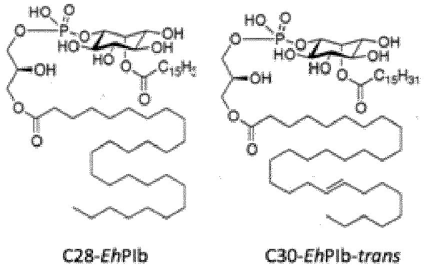 or one of the following structures:

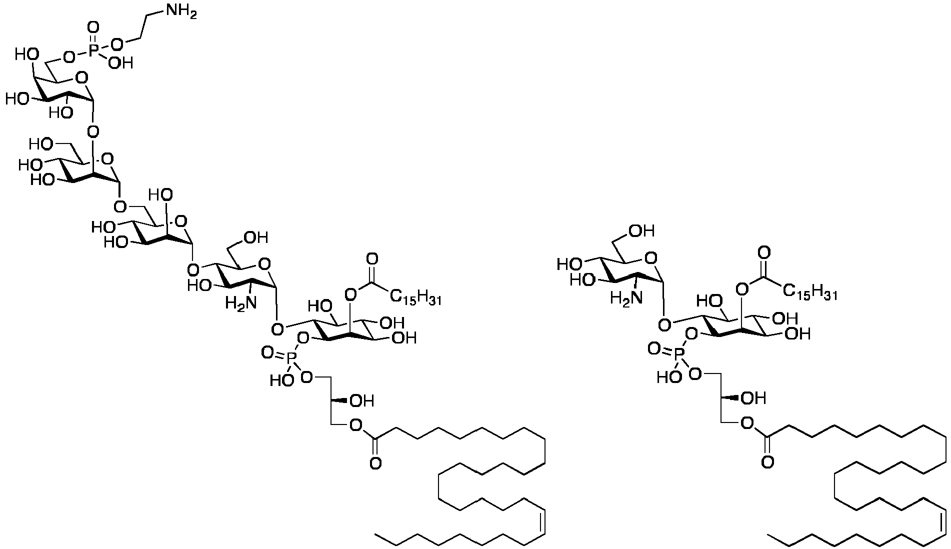

In still another aspect, the invention relates to a pharmaceutical composition comprising the above immunomodulatory compound.

### BRIEF DESCRIPTION OF THE FIGURES

Fig. 1 shows the intracellular production of cytokines IFN-γ, TNF-α, IL-17A and IL-4 in human NKT cells following stimulation with αGalCer and EhLPPG.
Fig. 2 shows the intracellular production of cytokines IFN-γ and IL-4 in human NKT cells following stimulation with αGalCer, EhLPPG, C30-EhPIa-cis and C30-EhPIb-cis. (A) Percentage of IFN-γ positive NKT cells. (B) Percentage of IL-4 positive NKT cells.
Fig. 3 shows the production of IFN-γ in murine spleen cells and sorted murine NKT cells following co-incubation with αGalCer, EhLPPG, C30-EhPIa-cis, C30-EhPIb-cis, C28-EhPIb and C30-EhPIb-trans pulsed BMDCs. (A) IFN-γ production in culture supernatant of stimulated spleen cells. (B) IFN-γ production in the culture supernatant of sorted murine NKT cells. IFN-γ was determined by ELISA.
Fig. 4 shows the results from liver toxicity measurements of EhLPPG und αGalCer in C57BL/6 mice. ALT levels measured in blood following four-time intraperitoneal injection of different amounts of αGalCer (A) und EhLPPG (B) are depicted.

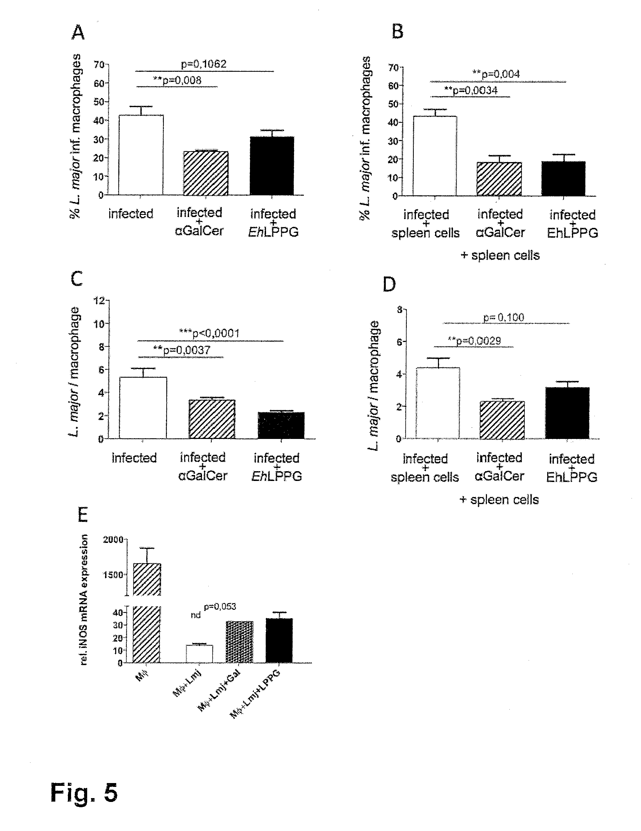

Fig. 5 shows the influence of EhLPPG and αGalCer on the infection of macrophages with Leishmania major in vitro. (A) Percentage of L. major infected macrophages following addition of EhLPPG and αGalCer (4 µg/ml) (8 µg/ml). (B) Percentage of *L. major* infected macrophages following addition of EhLPPG (8 µg/ml), αGalCer (4 µg/ml) and spleen cells. (C) Leishmania counts per macrophage following addition of EhLPPG and αGalCer. (D) Leishmania counts per macrophage following addition of EhLPPG (8 µg/ml), αGalCer (4 µg/ml) and spleen cells. (E) Influence of EhLPPG and αGalCer on the expression of iNOS-mRNA. MΦ = macrophages; (*) = significant difference to uninfected macrophages; p=0.053 value to untreated L. major infected macrophages (statistics: unpaired Students t-test).
Fig. 6 shows the influence of EhLPPG and αGalCer on the infection of mice with *L. major.* (A) Infection and application scheme. (B) Increase of foot-pad size over time after local injection of EhLPPG. (C) Ulceration-free survival time after local injection of EhLPPG.
Fig. 7 shows the influence of EhLPPG- or αGalCer-pulsed BMDCs on tumor development in a mouse model for mammary cancer. (A) Induction of mammary cancer and therapeutic application scheme using EhLPPG- or αGalCer-pulsed BMDCs. (B) Kaplan-Meier curve for determining the therapeutic influence of EhLPPG- or αGalCer-pulsed BMDCs on the tumor free survival time. (C) Influence of therapeutic application of EhLPPG- or αGalCer-pulsed BMDCs on tumor growth (statistics: Mantel Cox T test).
Fig: 8 to 15 are referred to in the synthesis part below.

### EXAMPLES

### Synthesis of EhPIa and EhPIb

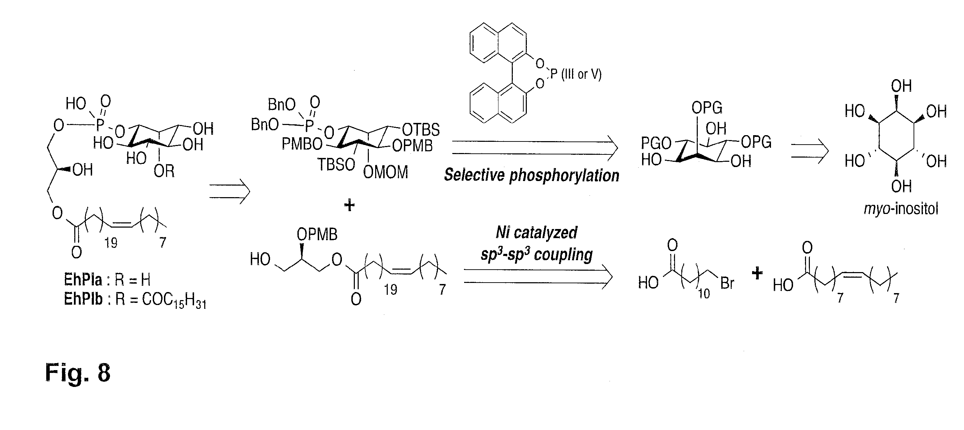

Following the scheme in Figure 8, the inositol phosphate part and the monoacyl glycerol part were independently prepared and both parts were linked together by using the Mitsunobu reaction similar to Xu et al. (2006). Acid removable protecting groups were used as semi-permanent protection for the hydroxy and phosphate groups, and the last step of the synthesis was their acidic cleavage. The inositol phosphate part was synthesized starting from the *myo*-inositol via regioselective phosphorylation of the inositol moiety with utilizing chiral phosphorylating reagents such as chiral binaphtol as chiral auxiliaries. C26 saturated fatty acid and C30 unsaturated fatty acids (30:1, *cis* and *trans*) were synthesized using a Ni catalyzed *sp³-sp³* cross coupling reaction described by Iwasaki, T. et al. (2013); Chem. Eur. J. 2013, 19, 2956-2960.

### Regioselective phosphorylation

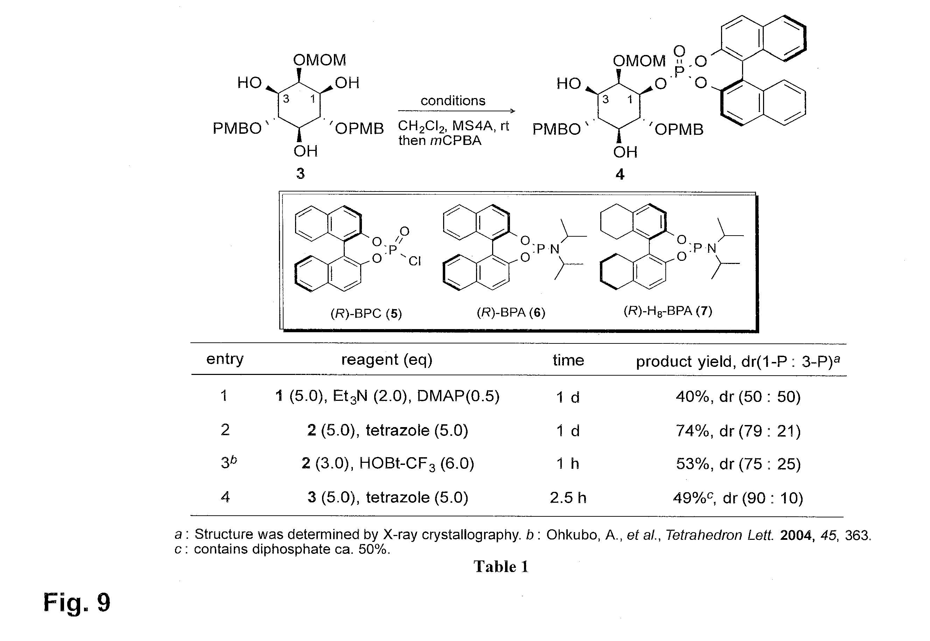

Regioselective phosphorylation was first investigated by using triol 3, which was derived from *myo-*inositol with 4 steps (Table 1 in Fig. 9). To obtain perfect selectivity for asymmetric phosphorylation of *myo*-inositol derivatives, and the separation of enantiomers, the regioselective phosphorylation was adopted using chiral phosphorylating reagents. The methods may afford a mixture of diastereomers, which can be readily separated.

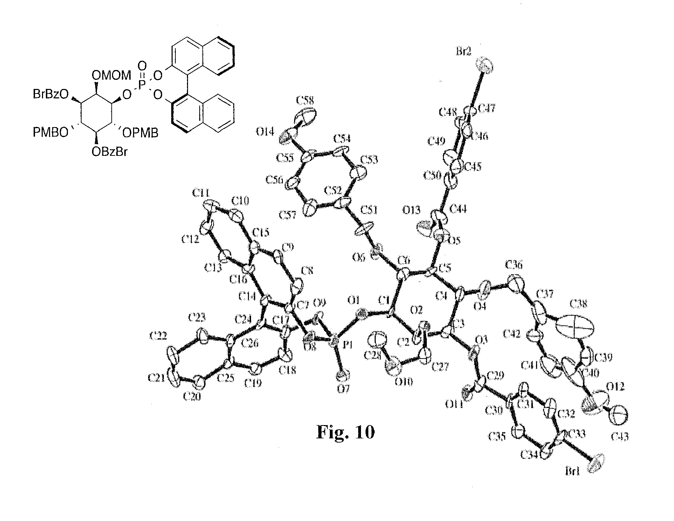

At first, *R*-BINOL was used as a chiral auxiliary. The phosphorylation using (*R*)-BPC (5) did not afford a good result in yield and selectivity (entry 1). Next, phosphoramidite method was used for phosphorylation. The reaction using (*R*)-BPA **(6)** in the presence of 1*H*-tetrazole as an activating reagent preferentially afforded the desired mono-phosphate **4** in 74% with regioselectivity 79:21 (desired 1-phosphate: undesired 3-phosphate) (entry 2). Reaction using highly reactive activating reagent HOBt-CF₃ decreased the yield (entry 3). Reaction with phosphorylating reagent 7 having octahydroBINOL as a chiral auxiliary also decreased the yield (entry 4). In both cases, the amounts of di- or tri-phosphorylating by-products were increased. The structure of the desired 1-phosphate was confirmed by the X-ray crystallographic analysis with using the *p*-bromobenzoylated derivative (see Fig. 10).

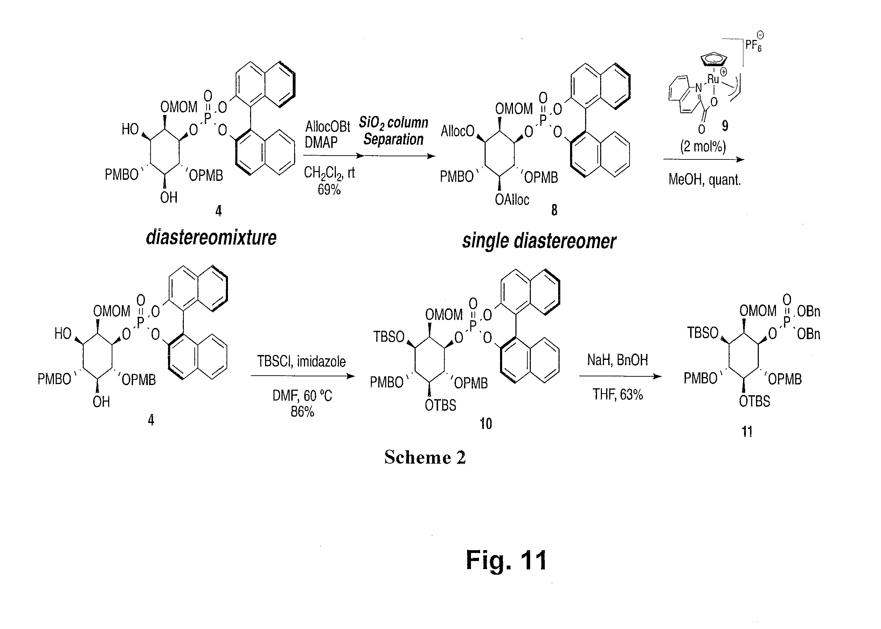

Product **4** was a diastereomeric mixture. After introduction of Alloc groups on two hydroxy groups in **4,** the desired **8** was readily obtained as a single diastereomer by simple silica-gel chromatography. After removal of Alloc groups of **8** by Ru-catalyst 9, Tanaka, S. et al. (2004), inositol phosphate ester 11 was synthesized via silylation and transesterification of BINOL to the benzyl ester (see Scheme 2 in Fig. 11).

### Synthesis of long-chain fatty acids using a sp³-sp³ coupling reaction

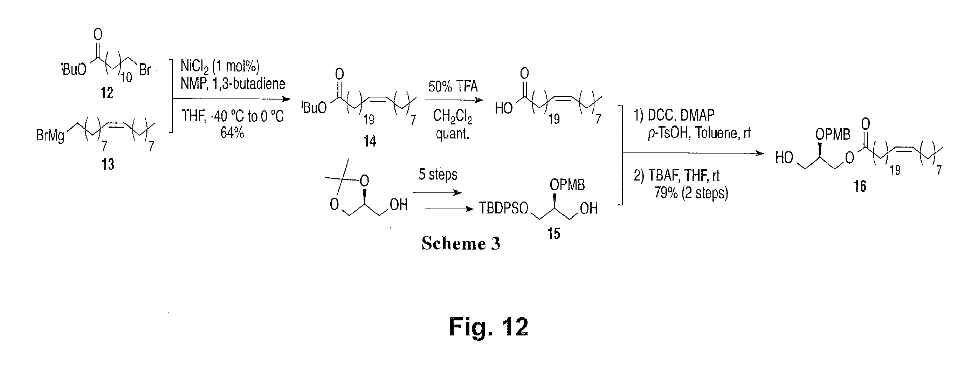

Long-chain fatty acids in EhPIa and EhPIa were synthesized according to the method shown in Scheme 3 in Fig. 12. The *sp³-sp³* coupling reaction of *t*-butyl ester of brominated fatty acid (C12) 12 with Grignard reagent 13 prepared from oleic acid (C18, *cis*) was carried out by using NiCl₂, 1, 3-butadiene, NMP in THF to give long-chain fatty acid *t*-butyl ester **14** having *cis*-double bond Iwasaki, T. et al. (2013) Chem. Eur. J., 19, 2956-2960. After removal of the *t*-butyl ester in **14** under acidic condition, the product was coupled with glycerol derivative **15** and the subsequent removal of the silyl group gave monoacyl glycerol **16.**

### Synthesis of C30-EhPIa-cis

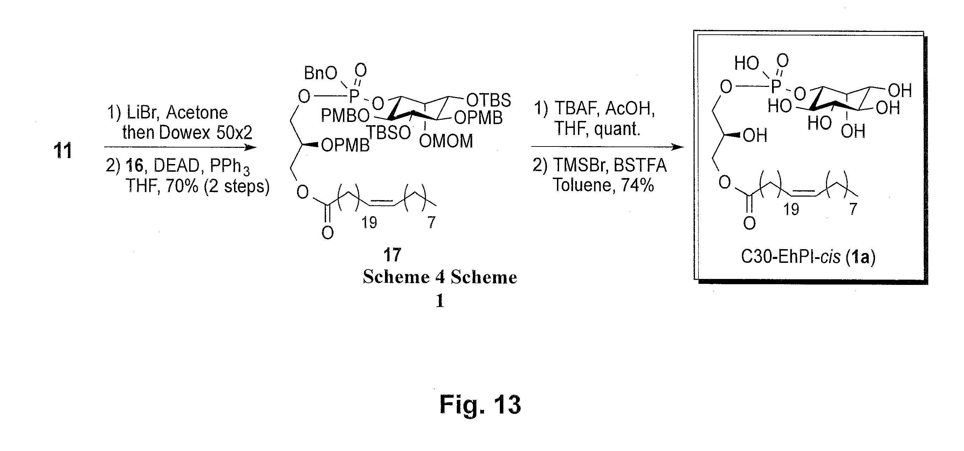

One of the benzyl groups of inositol phosphate ester **11** was then removed with LiBr. The diester obtained was then coupled with monoacyl glycerol **16** by using Mitsunobu reaction to afford 17. Finally, removal of silyl protecting groups with TBAF and then *p*-methoxybenzyl (PMB), methoxymethyl (MOM), and benzyl (Bn) groups gave the total synthesis of C30-EhPIa-*cis* (**1a**) (Scheme 4 in Fig. 13).

### Synthesis of EhPIb (2a, 2b, 2c) based on a new Allyl-protecting strategy

EhPIb, which shows selective cytokine induction, have a long-chain fatty acid and palmitic acid at 2-position on inositol. The selection of protecting groups on hydroxy functions therefore became more difficult. A new protecting group strategy was therefore employed for the synthesis of EhPIb. The cleavage of Allyl and Alloc groups on hydroxy functions using Ru catalyst **9** under mild neutral condition was reported previously. Here, a new strategy was developed in which Allyl and Alloc groups are used as semi-permanent protecting groups and Allyl and Alloc groups are simultaneously removed by using **9** at the final deprotection step.

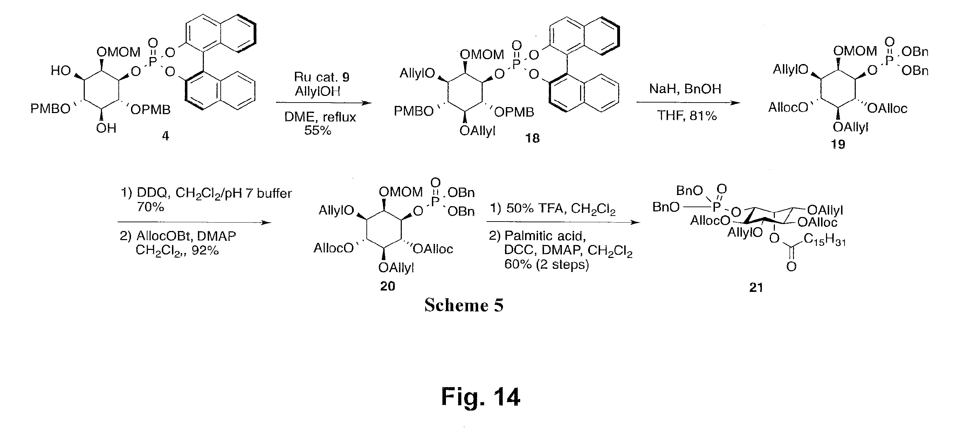

Ru-catalyzed dehydrative allylation (Saburi, H. et al. (2005) Angew. Chem. Int. Ed., 44, 1730-1732) of a common synthetic intermediate **4** using **9** gave di-allylated **18.** Transesterification of **18** afforded benzyl phosphate **19.** Selective removal of PMB with DDQ and subsequent introduction of Alloc groups to the liberated hydroxy groups yielded **20.** Afterwards, the 2-*O*-MOM group of **20** was cleaved by 50% TFA/CH₂Cl₂, and palmitic acid was introduced into the 2-position of the inositol moiety to give acylated inositol phosphate **21** (Scheme 5 in Fig. 14).

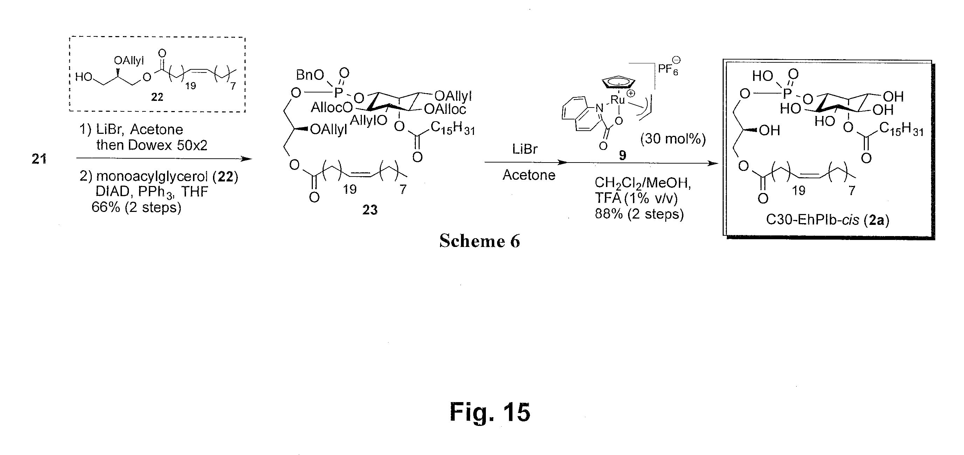

After removal of one of the benzyl groups on the phosphate in **21,** the resulting compound was coupled with Allyl-protected monoacyl glycerol derivative **22** under Mitsunobu conditions to give compound 23. After removal of the other benzyl group on the phosphate in **23,** final deprotection of all the allyl-type protecting groups was elucidated by using Ru catalyst **9.** Initially, the reaction was carried out under neutral conditions. Ally groups were, however, not cleaved at all, whereas Alloc groups were readily removed under neutral conditions. Lithium phosphate formed after the removal of benzyl group might quench the proton required for the activation of catalyst **9** and hamper the cleavage reaction of ally group. Final deprotection of all the allyl-type protecting groups of **23** was thus carried out by using Ru catalyst **9** in the presence of TFA to successfully afford the desired C30-EhPIb-*cis* **2a** in 88% yield. C30-EhPIb-*trans* **2b** and C28-EhPIb **2c** were also achieved in a similar manner (Scheme 6 in Fig. 15).

### Example 1: Measuring cytokine production in human NKT

This NKT cell assays uses whole PBMCs. The complete cells are activated by the stimulating compounds and FACS analysis allows detecting at the same time (a) the different NKT cell subpopulations and (b) intracellular cytokine production by these cells. This differs from the mouse NKT cell assay, where BMDCs are stimulated and then NKTs are added.

PBMCs from buffy coats or fresh blood samples from blood donors were used for intracellular cytokine analysis of NKT cells following stimulation with a phosphatidylinositol compound of the invention, for example the analogs C30-EhPIa-cis and C30-EhPIb-cis, by flow cytometry (Sandberg et al. 2003). Buffy coats for the isolation of PBMCs were kindly provided by the Department of Transfusion Medicine of the University clinic Hamburg-Eppendorf. All experiments were approved of by the ethical review committee of the Hamburger Ärztekammer (PV3551).

In short, blood was diluted 1:2 with PBS at room temperature (RT). Biocoll (Biochrom AG) was carefully overlayed with the blood/PBS mixture and centrifuged for 30 min, RT, 1500 rpm without break. After centrifugation the leukocyte ring was removed and transferred into a new falcon tube. Here, two fractions were pooled. The leukocytes were washed with sterile PBS and centrifuged for 20 min, 4°C and 1200 rpm. The supernatant was discarded, thrombocytes removed. Then, the cell pellets were once again pooled and washed with PBS for 12 min, 4°C, 1200 rpm. Subsequent to centrifugation, PBMCs were resuspended in 1 ml X-VIVO 15 supplemented with Pen/Strep (LONZA) or sterile PBS. PBMC were then used in the human iNKT cell assay.

For the human iNKT cell assay, a modified iNKT cell cytokine expression assay by Sandberg et al. (2003) was used. In brief, 1x10⁶ human PBMC/well were cultured in quadruplicates in 96-well round bottom plates with X-VIVOTM 15 supplemented with Pen/Strep (LONZA). Cells were pulsed with 1 µg/ml αGalCer, 10 µg/ml purified EhLPPG or 1-10 µg/ml synthetic EhPI anchors. As a co-stimulant, 3 µg/ml purified αCD28 was added per well for optimal iNKT cell activation without additional APC. Cells were incubated for 15h at 37°C, 5% CO₂. After one hour, 10 µg/ml Brefeldin A was added to the culture to stop golgi transport.

After incubation cells were harvested and iNKT cells were analyzed for their cytokine production of IFNγ-PE/Cy7, TNFα-FITC, IL-4-PE and IL-17A-BV421. iNKT cells were stained with CD3-PerCP and TCR Vα24-Jα18-APC (all antibodies were obtained by BioLegend). FACS was performed using a FACS LSRII device (BD Bioscience).

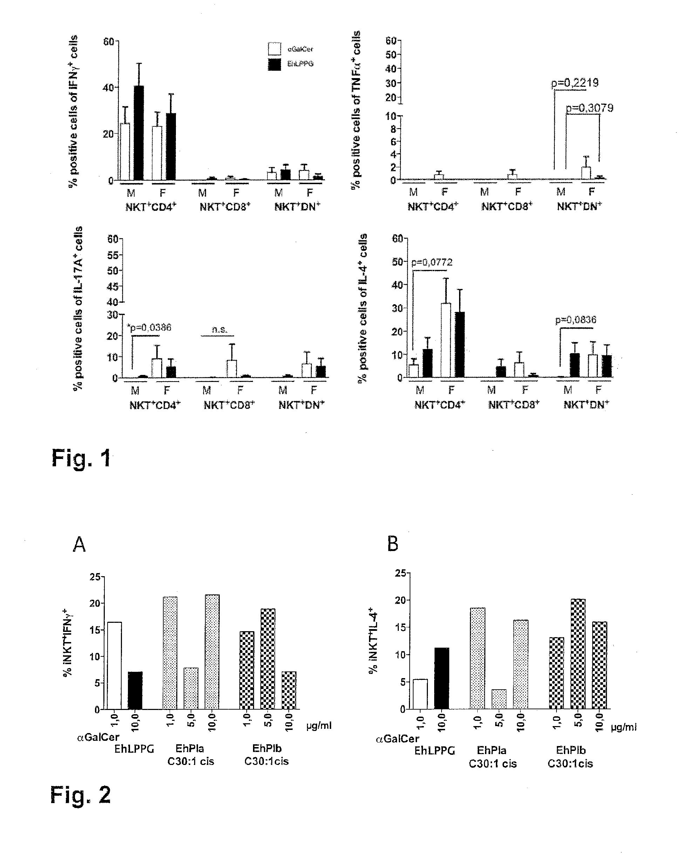

The results are shown in Figures 1 and 2. It can be seen that stimulation of human NKT cells with αGalCer or EhLPPG resulted in considerable amounts of CD4+NKT cells positive for IFN-γ and IL-4. However, in contrast to EhLPPG, αGalCer led to the production of tumor necrosis factor (TNF) α and IL-17, cytokines that may exhibit immunopathological functions when used for immunotherapeutic purposes (see Figure 1).

When using the two synthetic EhPI analogs C30-EhPIa-cis and C30-EhPIb-cis for stimulation, even higher percentages of CD4+NKT+IFNγ+ cells were obtained compared to stimulation with αGalCer. In contrast to αGalCer, and similar to EhLPPG, the synthetic analogs induced more CD4+NKT+IL-4+ cells compared to αGalCer (see Figure 2).

### Example 2: Measuring cytokine production in murine NKT

To test whether not only human cells, but also murine cells are stimulated by EhLPPG or the synthetic EhPI analogs, an NKT cell assay was performed with murine cells.

For the isolation of murine spleen cells 8 to 10-week-old mice were sacrificed and the spleens removed. The spleen was rinsed with RPMI 1640 (10% FCS, 1% L-Glutamine, 1% Sodium pyruvate, 50 µg/ml gentamycin) until it became translucent. The cell suspension was transferred to a falcon tube and centrifuged for 8 min, 4°C, 1200 rpm. Following centrifugation, cell pellets were adjusted to the appropriate number. For the isolation of T cells, spleen cells were subjected to magnetic bead sorting using the Pan T cell isolation kit II (MACS Miltenyi). Isolated T cells were directly used for the murine iNKT cell assay or for sorting of iNKT cells with αGalCer-CD1d-Tetramer-PE (kindly provided by the NIH-tetramer facility).

For cell sorting of murine iNKT cells, T cells isolated from the spleen as described above were stained with αGalCer-CD1d-Tetramer-PE for 30 min, 4°C. Washed twice with PBS at 1200 rpm, 4°C, 4 min and sorted with FACS AriaTM II (BD Biosciences).

BMDCs were used as antigen presenting cells (APC). In brief, 2-5x10⁴ cells/well were cultured in duplicates or triplicates in 96-well round bottom plates with RPMI 1640 (10% FCS, 1% L-Glutamine, 1% Sodium pyruvate, 50 µg/ml gentamycin). Cells were pulsed with 0.1-1 µg/ ml αGalCer, 20 µg/ml purified EhLPPG or 0.001-1 µg/ml synthetic EhPI anchors. iNKT enriched lymphocytes or sorted iNKT cells were then added to the pulsed APC with 2x10⁴-1x10⁵ cells/well and incubated for 48 h. IFN-γ was measured by ELISA (R&D Systems).

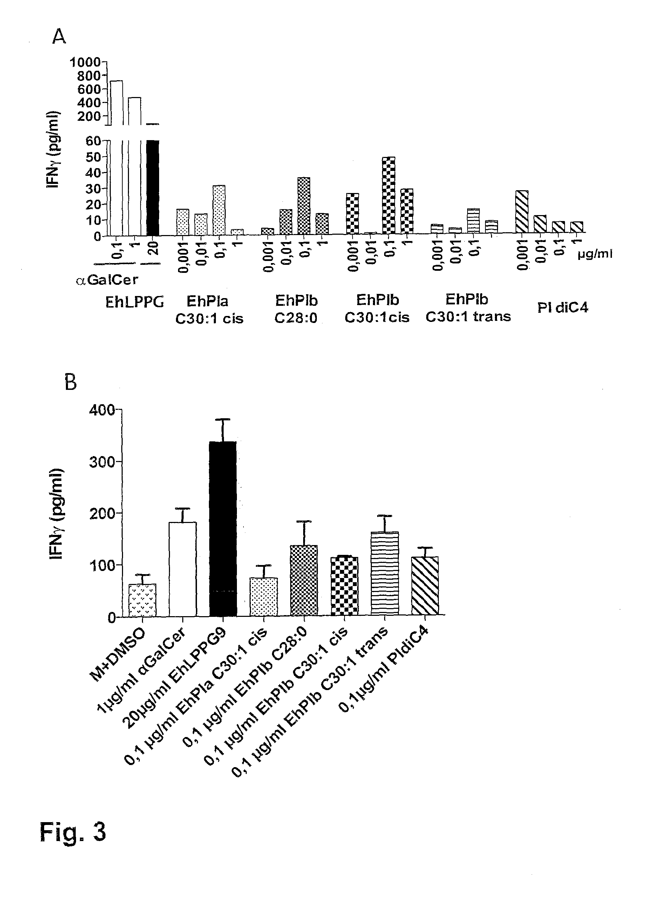

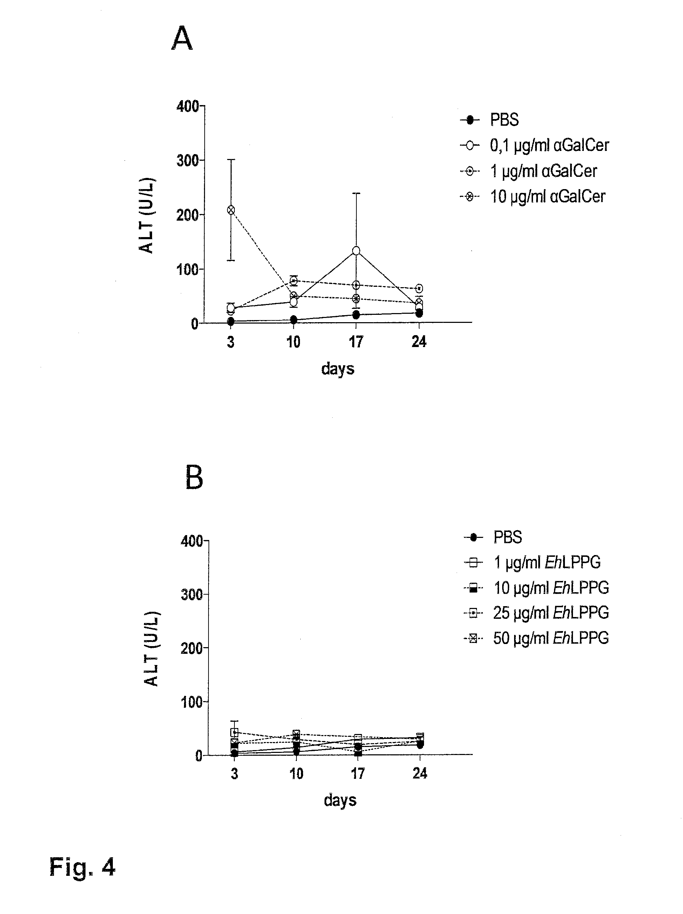

The results are shown in Figure 3. It was observed that also murine NKT cells are stimulated with the synthetic EhPI analogs. IFN-γ production was induced by all four synthetic analogs in spleen cell preparations in a dose dependent manner (see Figure 3A). IFN-γ production was likewise observed in sorted murine NKT cells (see Figure 4B). The results clearly demonstrate that the use of murine models is permissible to investigate the thepeutic potential of EhLPPG and its synthetic analogs.

### Example 3: Measuring liver toxicity

It was examined whether the intraperitoneal injections of EhLPPG and αGalCer are toxic for the liver of test animals. Different amounts of EhLPPG (1, 10, 25 and 50 µg/ml) and αGalCer (0.1, 1 and 10 µg/ml) were administered intraperitoneally into C57BL/6 mice. Subsequently, the alanine aminotransferase (ALT) levels were determined in the mouse serum (diluted 1:10 in H20) using the Cobas Integra® 400 plus Analyzer, Roche.

Figure 4 shows the results obtained from ALT determination. It can be seen that even high and frequent doses of EhLPPG do not induce an increase of the liver ALT enzyme in liver cells of mice. In contrast, a significant increase in the ALT levels was detected in mice that were treated with αGalCer.

### Example 4: In vitro Leishmania assay

It was investigated whether EhLPPG is able to influence intracellular infection of macrophages with *Leishmania major* parasites directly or indirectly by additionally adding immune cells to an *in vitro* infection assay.

Bone marrow derived macrophages (BMM) were harvested from tibias and femurs of 6 to 10 week-old female BALB/c mice and cultured for 10 days in Iscoves Modified Dulbecco's Medium (IMDM) supplemented with 10% FCS, 5% horse serum, 30% supernatant of L929 cells and antibiotics. In 8-well chamber slides (Nunc®) 2x10⁵ BMM/well were cultured in duplicates in IMDM without L929 cell supernatant and infected with promastigote *L. major* ASKH5 in a 1:10 ratio for 4h 37°C, 5% CO₂. After infection, BMMs were washed twice with warm PBS and 1 µg/ml αGalCer or 4 µg/ml purified EhLPPG was added with and without 4x10⁵ spleen cells in IMDM. The assay was incubated for 48 h, 37°C, 5% CO₂. Following incubation, cells were washed twice with warm PBS and stained with DAPI for calculating the parasite burden of the BMMs. BMMs were washed with wash buffer (1x PBS, 0.01 Triton-X-100), permeabilized (50 nM NH₄Cl, 1x PBS, 0.1% Triton-X-100) and stained for 1h, RT with 1 µg/ml DAPI diluted 1:50 in blocking solution (2% w/v BSA in 1x PBS, 0.1 % Triton-X-100). After staining, BMMs were washed with wash buffer and a cover slip was fixed with Mowiol (25% glycerol, 0.1 M Tris-HCl (pH 8.5), 10% w/v Mowiol 4-88) on the chamber slides. Parasite burden was estimated by counting kinetoplasts per macrophage.

As shown in Fig. 5, it was found that both EhLPPG and αGalCer significantly influenced the intracellular *L. major* infection rate. Addition of EhLPPG to the culture led to a considerable reduction in the percentage of infected macrophages (see Figure 5A) that became significant when spleen-derived lymphocytes were co-incubated (see Figure 5B). The percentage of infected macrophages was significantly reduced in both cases following treatment of the cell culture with αGalCer (see Figures 5A and 5B).

Regarding the influence of EhLPPG treatment on the parasite load per macrophage, it was found that treatment with EhLPPG, as with αGalCer, significantly reduced the amount of parasites within the macrophages (see Figure 5C). Addition of spleen cells led to reduction in the amounts of intracellular parasites when EhLPPG was added to the culture and a significant reduction when αGalCer was added to the culture (see Figure 5D). From the literature it is known that the inducible nitric oxide synthase (iNOS) plays a crucial role in the growth-control of intracellular *Leishmania* parasites. Preliminary results now suggest that EhLPPG and presumably also αGalCer lead to increase in the expression of iNOS thus leading to a decrease in the parasite load of macrophages (data not shown).

### Example 5: Animal model for cutaneous leishmaniasis

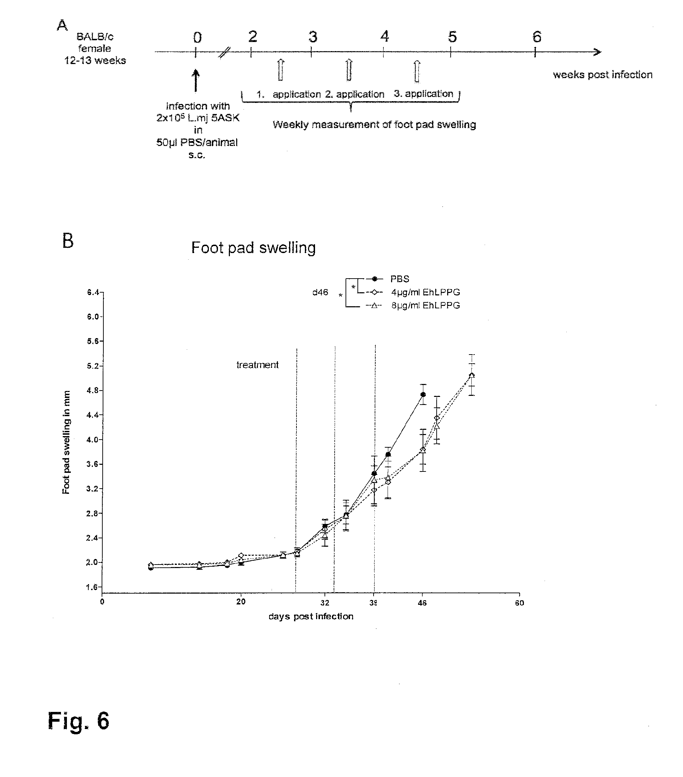

The influence of EhLPPG and αGalCer treatment was investigated in an animal model for cutaneous leishmaniasis. The infection and application scheme is shown in Figure 6A. 10 to 14-week old female BALB/c mice were infected subcutaneously (s.c.) into the foot pad of the right hind leg with 2x10⁵ promastigote *L. major* ASKH5 in 50 µl PBS. With developing foot pad swelling, mice were treated s.c. once a week with either 4 µg/ml or 8 µg/ml purified EhLPPG in 25 µl PBS or with PBS only as a control. Foot pad swelling was estimated twice a week with a caliper. According to the guidelines of the ethic commission, mice were sacrificed at the time point of lesion ulceration.

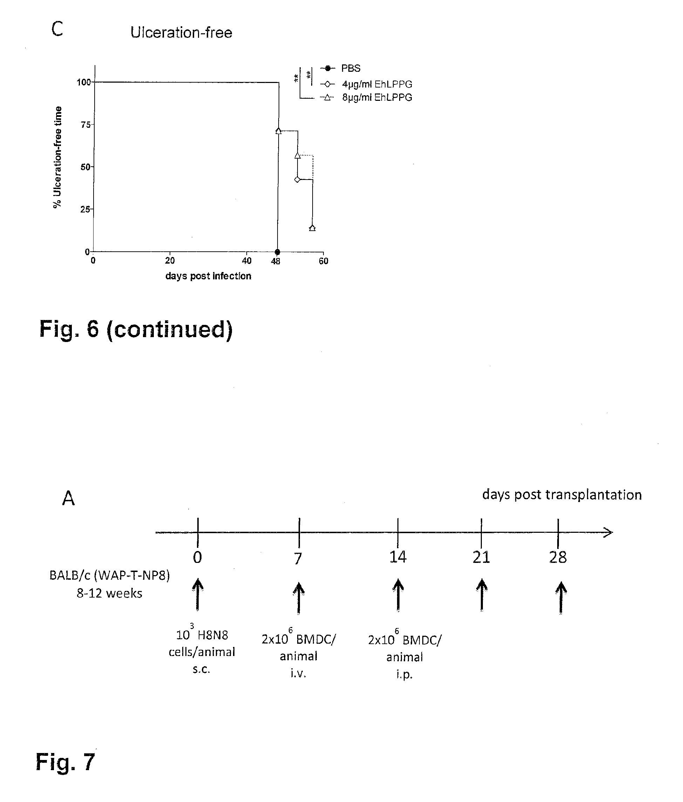

The results are shown in Figure 6B and 6C. Beginning from the second application on, treated mice showed a significant (4 µg/ml: p<0.05, 8 µg/ml: p<0.05) reduction and deceleration in the size of the footpads that became significantly different from PBS treated mice at day 46 after infection. At this time point, all PBS treated mice had to be sacrificed due to ulceration of *Leishmania* lesions. In the following, mice treated with 4 or 8 µg/ml purified EhLPPG showed a continuous increase in the size of the footpads without signs of ulceration. Figure 6C shows that the onset of the footpad swelling was significantly delayed in EhLPPG treated mice (4 µg/ml: p<0.005, 8 µg/ml: p<0.005) compared to PBS treated control mice.

### Example 6: Animal model for breast cancer

It was examined whether EhLPPG- or αGalCer-pulsed BMDCs could influence tumor growth in a mouse model for mammary cancer. Female BALB/c mice (WAP-T-NP8) were injected with the tumor cell line H8N8 resulting which induces the development of a singular tumor within 4-6 weeks. See Wegwitz et al., 2010. Determination of a therapeutic effect can be estimated by the time of the tumor free survival as well as growth rate and size of a tumor. The treatment scheme is shown in Figure 7A. The mice were injected intraperitoneally with BMDCs pre-incubated for 3 hours with either 4 µg EhLPPG/ml or 1 µg αGalCer/ml on day 7 and 14 post implantation. As control, two groups of mice received either PBS or BMDCs alone (Figure 7A).

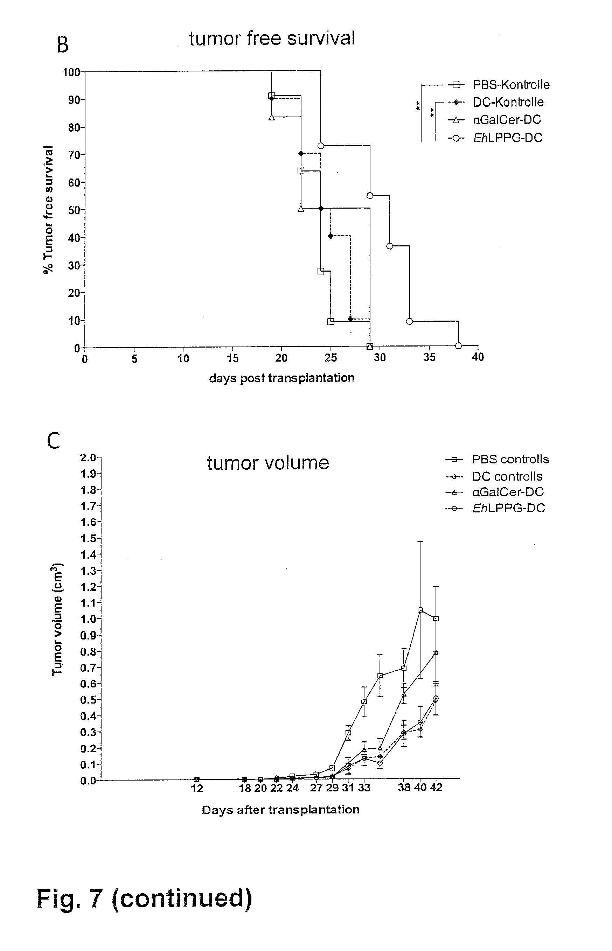

The results are shown in Figure 7B. It can be seen that the treatment with EhLPPG-pulsed BMDCs, but not αGalCer-pulsed BMDCs, significantly increased the tumor free survival time compared to mice that had received PBS or BMDCs alone. Furthermore, the tumor growth was significantly reduced in mice that had been treated with EhLPPG-pulsed BMDCs compared to PBS controls within the first 7 days following a second therapeutic intervention. However, treatment with BMDCs alone also resulted in a decrease of the tumor growth within the same period. At later time points, again no difference in the tumor growth was observed between EhLPPG-pulsed and non-stimulated BMDCs but the tumor sizes were reduced compared to PBS treated controls. Most interestingly, treatment with αGalCer-pulsed BMDCs initially reduced the tumor growth in comparison to PBS controls but tumor growth was enhanced compared to EhLPPG-pulsed BMDCs and BMDCs at later time points (Figure 7C).

### LITERATURE

Arias JL (2013), Liposomes in drug delivery: a patent review (2007-present), Expert Opin Ther Pat. 23(11):1399-414.
Barros NB, et al. (2013) Liposomal-lupane system as alternative chemotherapy against cutaneous leishmaniasis: macrophage as target cell. Experimental parasitology 135: 337-343.
Blum J, et al. (2012) Local or systemic treatment for New World cutaneous leishmaniasis? Re-evaluating the evidence for the risk of mucosal leishmaniasis. International health 4: 153-163.
Ishikawa A, et al. (2005) A phase I study of α-galactosylceramide (KRN-7000)-pulsed dendritic cells in patients with advanced and recurrent non-small cell lung cancer. Clinical Cancer Research 11 (5): 1919-7.
Iwasaki, T. et al. (2013); Chem. Eur. J. 19, 2956-2960.
Laurent X, et al. (2014) Switching Invariant Natural Killer T (iNKT) Cell Response from Anti-cancerous to Anti-Inflammatory Effect: Molecular Bases. Journal of medicinal chemistry 57: 5489-5508.
Lotter H, et al. (2009), Natural killer T cells activated by a lipopeptidephosphoglycan from Entamoeba histolytica are critically important to control amebic liver abscess. PLoS Pathog 5: e1000434.
Lotter H, et al. (2013), Testosterone increases susceptibility to amebic liver abscess in mice and mediates inhibition of IFNgamma secretion in natural killer T cells. PloS one 8: e55694.
Neumayr AL, et al. (2013) Clinical aspects and management of cutaneous leishmaniasis in rheumatoid patients treated with TNF-alpha antagonists. Travel medicine and infectious disease 11: 412-420.
Sandberg JK, et al. (2003) Dominant effector memory characteristics, capacity for dynamic adaptive expansion, and sex bias in the innate Valpha24NKT cell compartment. Eur J Immunol 33(3): 588-96.
Sosa N, et al. (2013) Randomized, double-blinded, phase 2 trial of WR 279,396 (paromomycin and gentamicin) for cutaneous leishmaniasis in Panama. The American journal of tropical medicine and hygiene 89: 557-563.
Tanaka, S. et al. (2004) Org. Lett., 4, 1873-1875.
Uchida T, et al. (2008) Phase I study of alpha-galactosylceramide-pulsed antigen presenting cells administration to the nasal submucosa in unresectable or recurrent head and neck cancer, Cancer Immunol. Immmunother. 57: 337-345.
Wegwitz F, et al. (2010) Tumorigenic WAP-T mouse mammary carcinoma cells: a model for a self-reproducing homeostatic cancer cell system. PloS one 5: e12103.
Xu, YJ et al (2006) J. Org. Chem., 71, 4919-4928.

## Claims

1. Compound having the structure of the following formula (I): 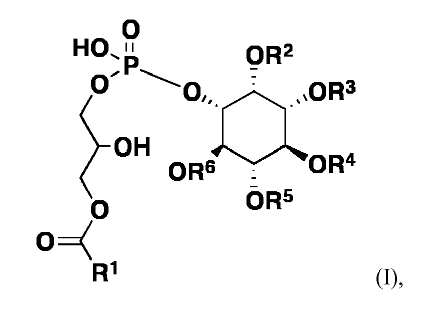 wherein
R¹ is Y or Z,
R², R³ and R⁵ are independently selected from -H, -C(O)Y, -C(O)Z, and groups of formula (II) 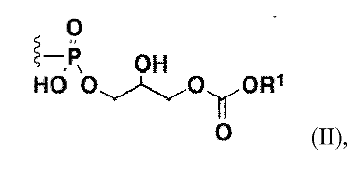 and
R⁴ and R⁶ are independently selected from -H, -C(O)Y, -C(O)Z, and groups derived from glycosides,
wherein
Y is CH₃(CH₂)ₘ-, and m = 0-35, and
Z is CH₃(CH₂)ₙ-(CH=CH)ₚ(CH₂)_{q}-, and n and q are independently 0-35, and p = 1-4
or a pharmaceutically acceptable salt, solvate, prodrug, and protected derivative thereof,
for use in a method of treating a cancer disease or an infection with an intracellular pathogen in a subject in need thereof.

2. Compound for use in a method of claim 1, wherein said subject is a human.

3. Compound for use in a method of any of claims 1-2, wherein said cancer disease is selected from the group of breast cancer, colon cancer, pancreatic cancer, stomach cancer, brain cancer, lung cancer, kidney cancer, liver cancer, ovarian cancer, cervical cancer, or thymus cancer.

4. Compound for use in a method of claim 3, wherein said cancer is breast cancer, preferably a carcinoma selected from the group of invasive ductal carcinoma, invasive lobular carcinoma, papillary carcinoma, mucinous carcinoma, medullary carcinoma and tubular carcinoma.

5. Compound for use in a method of any of claims 1-2, wherein said infection with an intracellular pathogen is an infection with a bacterial or protozoan microorganism

6. Compound for use in a method of claim 5, wherein said microorganism is a bacterium from the genus Mycobacterium, Rickettsia, Brucella, Chlamydia, Listeria, Legionella, Coxiella, Francisella, or a protozoa from the genus Leishmania.

7. Compound for use in a method of claim 6, wherein said method is for treating cutaneous leishmaniasis.

8. Compound for use in a method of any of claims 1-7, wherein said compound is selected from the group of: 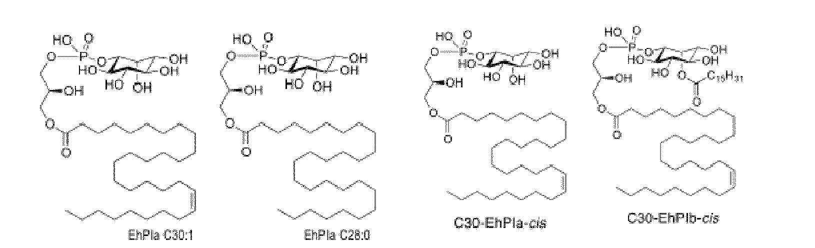 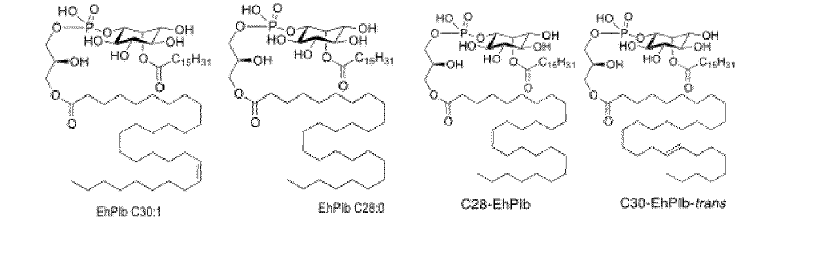 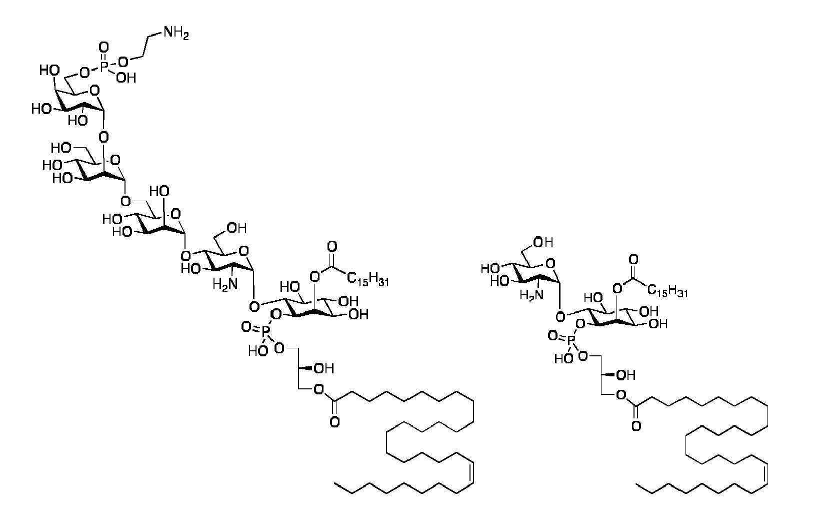 or a pharmaceutically acceptable salt, solvate, prodrug, and protected derivative thereof

9. Compound for use in a method of any of claims 1-8, wherein the treatment comprises the ex vivo pre-incubation of the compound with antigen presenting cells (APCs) of the subject and the subsequent re-infusion of the APCs into the subject.

10. Method for preparing a composition for inducing and/or enhancing an immune response, said method comprising
(a) separating CD14+ monocytes from a blood sample obtained from a cancer, preferably a breast cancer patient, patient;
(b) differentiating said CD14+ monocytes into APCs;
(c) incubating the APCs with a compound as defined in claim 1;
(d) optionally, purifying the APCs.

11. Method of claim 10, wherein said CD14+ monocytes are differentiated into dendritic cells (DCs) by the addition of IL-4 and/or GM-CSF.

12. Method of any of claims 10-11, wherein said incubation in step (c) is performed for a period of 2-5 hours.

13. Method of any of claims 10-12, wherein the incubation in step c) is performed with 10-100 µg/ml of said compound.

14. Compound selected from the group of: 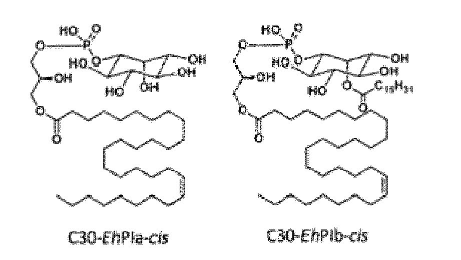 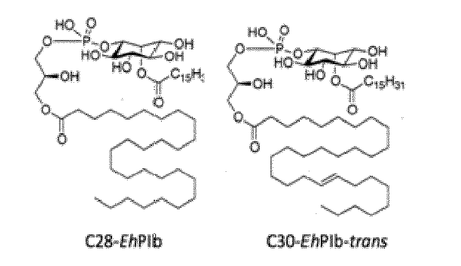 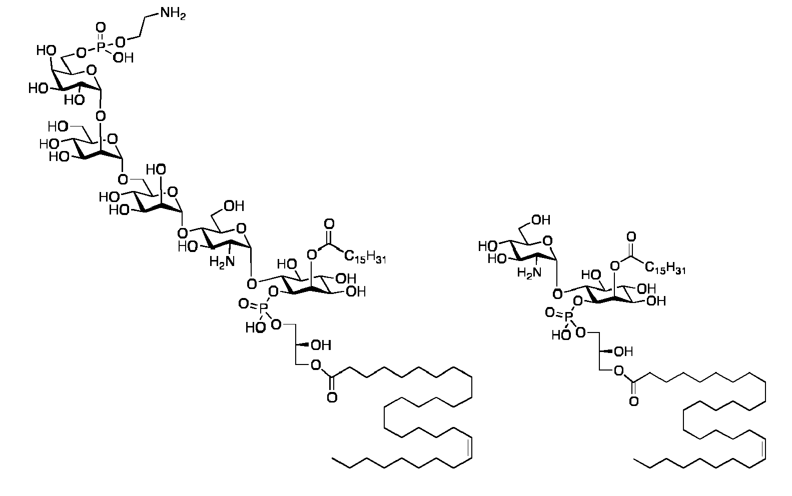 or a pharmaceutically acceptable salt, solvate, prodrug, and protected derivative thereof.

15. Pharmaceutical composition comprising a compound selected from the group of: 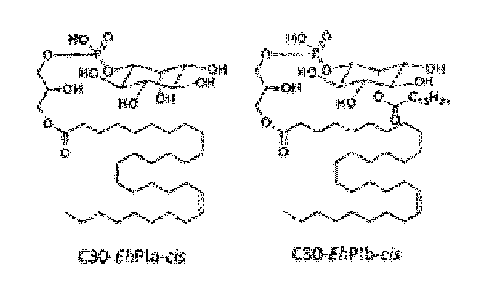 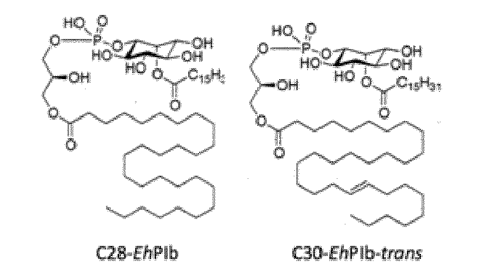 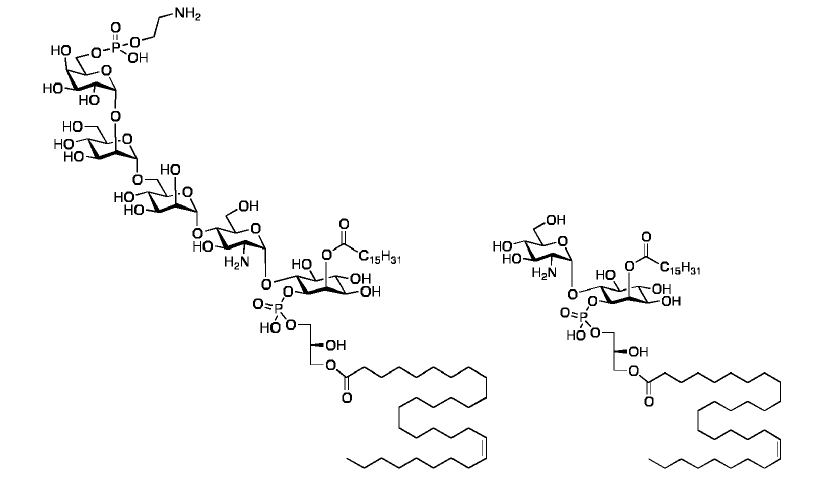 or a pharmaceutically acceptable salt, solvate, prodrug, and protected derivative thereof.
